# EUROPEAN PATENT APPLICATION

(11) **EP 4 368 250 A2**
(43) Date of publication of application: **15.05.2024**
(21) Application number: 24150094.1
(22) Date of filing: 04.03.2022
(51) Int. Cl.: A61P 25/28

(54) **OBICETRAPIB FOR TREATMENT OF DEMENTIAS**

(30) Priority: 05.03.2021 US 202163157586 P
(62) Divisional of application: 22715162.8
(71) Applicant: NewAmsterdam Pharma B.V., 1411 DC Naarden (NL)
(72) Inventor: Ditmarsch, Marc, 1411 DC Naarden (NL); Kastelein, Johannes Jacob Pieter, 1411 DC Naarden (NL); Davidson, Michael Harvey, 1411 DC Naarden (NL)
(74) Representative: Nederlandsch Octrooibureau

(57) **Abstract**

Methods are presented for slowing progression of neurodegenerative disease in a subject who has or is at risk of developing a neurodegenerative disease. The methods comprise administering a therapeutically effective amount of obicetrapib or a pharmaceutically acceptable salt thereof to the subject.

## Description

### 1. CROSS-REFERENCE TO RELATED APPLICATIONS

This application claims the benefit of and priority to US provisional application no. 63/157,586 filed March 5, 2021, the disclosure of which is incorporated herein by reference in its entirety.

### 2. BACKGROUND OF THE INVENTION

Neurodegenerative diseases, and particularly neurodegenerative diseases that result in cognitive impairment and dementia, are a serious burden on patients, their families, and society. Alzheimer's Disease and other dementias have been calculated to have cost 2.55 million disability-adjusted life-years (DALYs) in the United States in 2017. *See* "Burden of neurological disorders across the US from 1990-2017," JAMA Neurol. 78(2): 165-176 (2021). The burden is expected to increase as the median age of the population increases.

Despite the need for effective treatments, no disease-modifying treatments capable of slowing progression of neurodegenerative diseases, particularly degenerative diseases that result in cognitive impairment and dementia, have been demonstrated to be effective. In addition, patients with cognitive impairment present compliance challenges for treatments that must be administered chronically, and/or administered using a patient-controlled device. Treatments that require parenteral administration impose additional impediments. There is, therefore, a need for disease-modifying treatments capable of slowing progression of neurodegenerative diseases, particularly degenerative diseases that result in cognitive impairment and dementias, and that can be administered orally.

### 3. SUMMARY OF THE INVENTION

The ε4 allele of the apolipoprotein E gene (APOE4) has long been known to be a risk factor for late-onset Alzheimer's disease (AD). Although the mechanism by which this genotype increases AD risk remains unclear, the strength of this association positions lipoprotein metabolism as central to the pathology of AD.

Obicetrapib, a potent inhibitor of cholesterol esterase transfer protein (CETP), has previously been shown to increase HDL levels and ApoE levels in plasma. We have now shown that obicetrapib crosses the blood-brain barrier, with measurable CNS accumulation following oral administration. Using a transgenic mouse model of Alzheimer's disease that has been further engineered to express human CETP, we demonstrate that obicetrapib reduces deterioration in performance on a cognitive test, and reduces concentrations of Aβ fragments in the soluble fraction of the brain.

Accordingly, a method is provided for slowing progression of neurodegenerative disease in a subject who has or is at risk of developing a neurodegenerative disease. The method comprises administering a therapeutically effective amount of obicetrapib or a pharmaceutically acceptable salt thereof to the subject.

In some embodiments, obicetrapib or salt thereof is administered orally. In various embodiments, obicetrapib is administered once per day. In certain embodiments, the dose is 5-25 mg po QD of obicetrapib or salt thereof, such as 5 mg po QD, 10 mg po QD, 15 mg po QD, 20 mg po QD, or 25 mg po QD.

In some embodiments, obicetrapib or salt thereof is administered once daily for at least 8 weeks, at least 6 months, at least 12 months, or at least 24 months.

In some embodiments, the subject has been diagnosed with or is at risk for Alzheimer's Disease (AD), Lewy Body dementia, vascular or multi-infarct dementia, or frontotemporal dementia (FTD). In particular, embodiments, the subject has been diagnosed with or is at risk for developing AD. In specific embodiments, the subject has been diagnosed with AD. In certain embodiments, the subject has at least one ApoE4 allele or two ApoE4 alleles. In specific embodiments, the method comprises the prior step of detecting ApoE4 alleles in the subject.

In some embodiments, the subject has been diagnosed with mild cognitive impairment (MCI).

In some embodiments, the subject has been determined, prior to treatment, to have at least one of (i) decreased levels of Aβ42 in plasma, (ii) increased levels of Aβ40 in plasma, (iii) decreased ratio of Aβ42/Aβ40 in plasma, (iv) increased levels of neurofilament light (NfL) in plasma, and (v) increased levels of neurogranin in plasma, as compared to a healthy control population that does not have and that is not at elevated risk for neurodegenerative disease.

In some embodiments, the subject has been determined, prior to treatment, to have at least one of (i) decreased levels of Aβ42 in CSF, (ii) increased levels of Aβ40 in CSF, (iii) decreased ratio of Aβ42/Aβ40 in CSF, (iv) increased levels of neurofilament light (NFL) in CSF, and (v) increased levels of neurogranin in CSF, as compared to a healthy control population that does not have and that is not at elevated risk for neurodegenerative disease.

In some embodiments, the subject has been determined, prior to treatment, to have abnormal patterns of Tau based on Tau PET imaging.

In some embodiments, the dose of obicetrapib or salt thereof is sufficient to prevent at least one of (i) further decrease in levels of Aβ42 in plasma, (ii) further increase in levels of Aβ40 in plasma, (iii) further decrease in ratio of Aβ42/Aβ40 in plasma, (iv) further increase in levels of neurofilament light (NFL) in plasma, and (v) further increase in levels of neurogranin in plasma, as compared to levels immediately prior to start of treatment.

In some embodiments, the dose of obicetrapib or salt thereof is sufficient to prevent at least one of (i) further decrease in levels of Aβ42 in CSF, (ii) further increase in levels of Aβ40 in CSF, (iii) further decrease in ratio of Aβ42/Aβ40 in CSF, (iv) further increase in levels of neurofilament light (NFL) in CSF, and (v) further increase in levels of neurogranin in CSF, as compared to levels immediately prior to start of treatment.

In typical embodiments, obicetrapib is administered as a tablet. In certain embodiments, the tablet comprises obicetrapib as the calcium salt.

### 4. BRIEF DESCRIPTION OF THE DRAWINGS

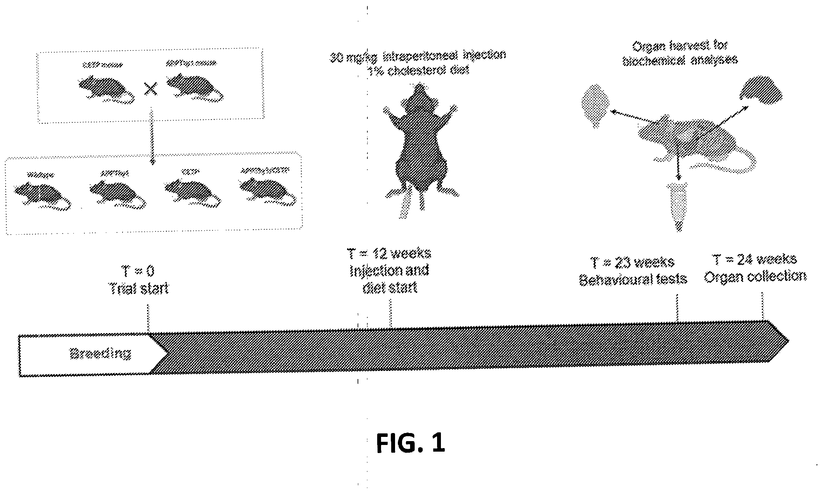

**FIG.1** is visual description of the timeline of the transgenic mouse experiment.

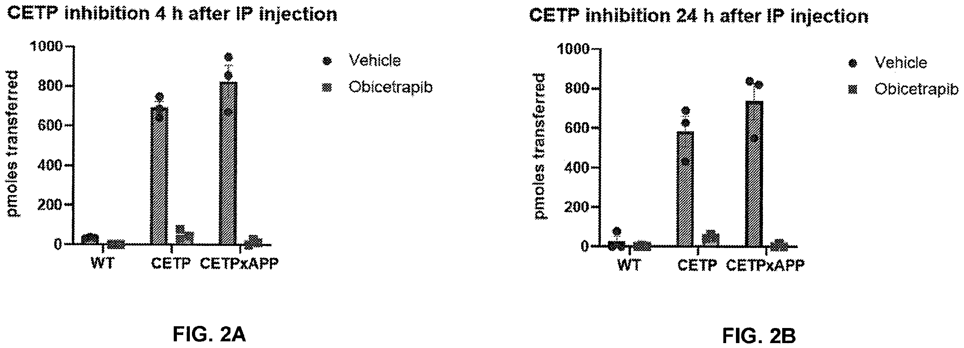

**FIGS. 2A** and **2B** are bar graphs showing that CETP transgenic mice and hCETP/APP (amyloid precursor protein) double transgenic mice produced human CETP, and the effect of intraperitoneally administered 30 mg/kg obicetrapib on CETP activity in these transgenic mice. The data indicate hat the transgenic mice on vehicle injections exhibit CETP activity as compared to wild type mice, which lack CETP. The data also show that CETP activity was inhibited in the obicetrapib treatment group.

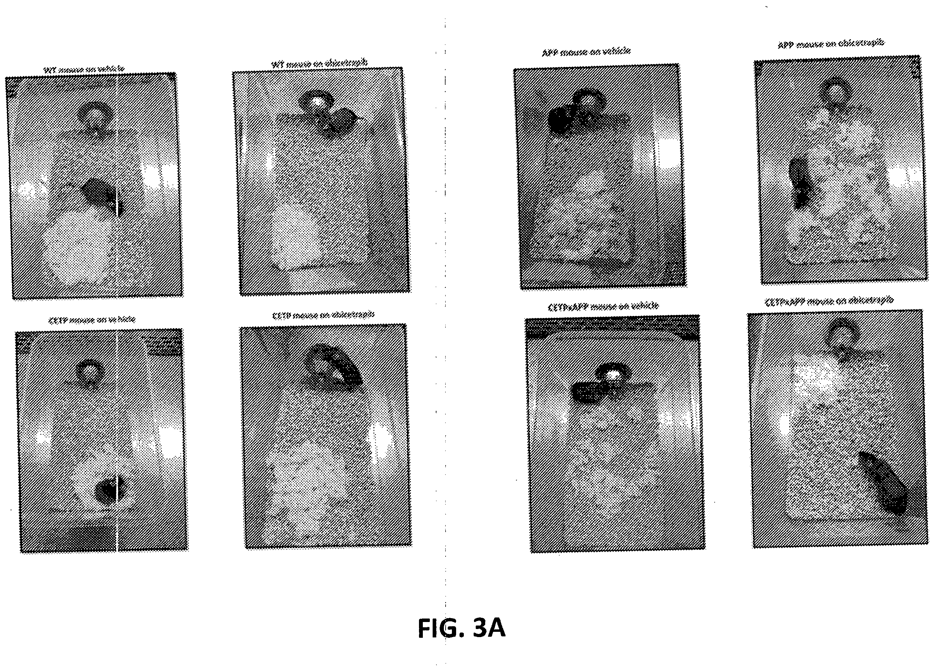

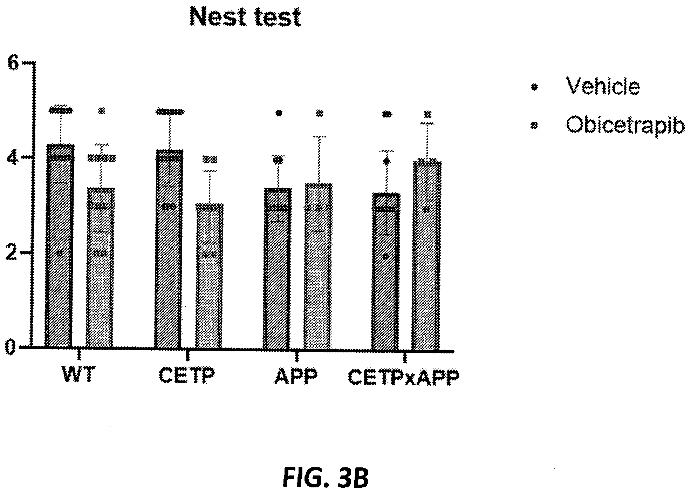

FIGS. 3A-B provide results from a nest building cognitive performance test. FIG. 3A is visual display of the effect on nest building in mice under various conditions. FIG. 3B is a bar graph quantifying on a 5-point scale the nest building ability imaged in FIG. 3A. The data indicate that the hCETP/APP transgenic mice fed a cholesterol-containing diet showed decreased performance on this cognitive test when treated with vehicle (negative control), and that administration of obicetrapib decreases this age-related cognitive degradation.

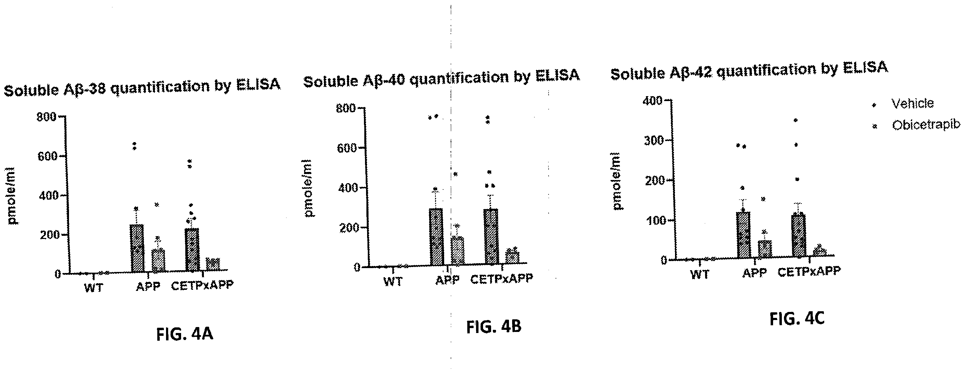

FIGs. 4A-C show bar graphs quantifying amyloid-beta (Aβ) peptides found in TBS-soluble brain extracts analyzed using ELISA. The data indicate that obicetrapib decreases Aβ production especially in the HCETP/APP double transgenic group. APP transgenic mice also show decreased Aβ production.

### 5. DETAILED DESCRIPTION OF THE INVENTION

### 5.1. Definitions

Unless defined otherwise, all technical and scientific terms used herein have the meaning commonly understood by a person skilled in the art to which this invention belongs.

The terms "**subject**" or "**individual**" are used interchangeably and refer to an animal to be treated, including but not limited to humans and non-human primates; rodents, including rats and mice; bovines; equines; ovines; felines; and canines.

The term "**patient**" refers to a human subject.

The terms "**treating**", "**treatment**", and grammatical variations thereof are used in the broadest sense understood in the clinical arts. Accordingly, the terms do not require cure or complete remission of disease, and encompass obtaining any clinically desired pharmacologic and/or physiologic effect.

The phrase "**therapeutically effective amount**" refers to the amount of a compound that, when administered to a subject for treating a disease, condition, or disorder, is sufficient to effect treatment of the disease, condition, or disorder.

The term "**pharmaceutically acceptable salt**" refers to a salt that is acceptable for administration to a subject. Examples of pharmaceutically acceptable salts include, but are not limited to: mineral acid salts such as hydrochloride, hydrobromide, hydroiodide, phosphate, sulfate, and nitrate; sulfonic acid salts such as methanesulfonate, ethanesulfonate, benzenesulfonate, p-toluenesulfonate, and trifluoromethanesulfonate; organic acid salts such as oxalate, tartrate, citrate, maleate, succinate, acetate, trifluoroacetate, benzoate, mandelate, ascorbate, lactate, gluconate, and malate; amino acid salts such as glylcine salt, lysine salt, arginine salt, ornithine salt, glutamate, and aspartate; inorganic salts such as lithium salt, sodium salt, potassium salt, calcium salt, and magnesium salt; and salts with organic bases such as ammonium salt, triethylamine salt, diisopropylamine salt, and cyclohexylamine salt. The term "salt(s)" as used herein encompass hydrate salt(s). Other examples of pharmaceutically salts include anions of the compounds of the present disclosure compounded with a suitable cation.

### 5.2. Other interpretational conventions

Ranges: throughout this disclosure, various aspects of the invention are presented in a range format. Ranges include the recited endpoints. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6, should be considered to have specifically disclosed subranges such as from 1 to 3, from 1 to 4, from 1 to 5, from 2 to 4, from 2 to 6, from 3 to 6, etc. as well as individual number within that range, for example, 1, 2, 3, 4, 5, 5.3, and 6. This applies regardless of the breadth of the range.

In this disclosure, "comprises", "comprising", "containing", "having", "includes", "including" and linguistic variants thereof have the meaning ascribed to them in U.S. Patent law, permitting the presence of additional components beyond those explicitly recited.

Unless specifically stated or apparent from context, as used herein the term "or" is understood to be inclusive.

Unless specifically stated or apparent from context, as used herein, the terms "a", "an", and "the" are understood to be singular or plural. That is, the articles "a" and "an" are used herein to refer to one or to more than one (*i.e.*, to at least one) of the grammatical object of the article. By way of example, "an element" means one element or more than one element.

Unless specifically stated or otherwise apparent from context, as used herein the term "about" is understood as within range of normal tolerance in the art. Unless otherwise specified, "about" intends ±10% of the stated value. Where a percentage is provided with respect to an amount of a component or material in a composition, the percentage should be understood to be a percentage based on weight, unless otherwise stated or understood from the context.

Unless the specific stereochemistry is expressly indicated, all chiral, diastereomeric, and racemic forms of a compound are intended. Thus, compounds described herein include enriched or resolved optical isomers at any or all asymmetric atoms as are apparent from the depictions. Racemic mixtures of R-enantiomer and S-enantiomer, and enantio-enriched stereomeric mixtures comprising of R- and S-enantiomers, as well as the individual optical isomers can be isolated or synthesized so as to be substantially free of their enantiomeric or diastereomeric partners, and these stereoisomers are all within the scope of the present technology.

### 5.3, Summary of experimental observations

Obicetrapib, formerly known as TA-8995, is a potent inhibitor of cholesterol ester transfer protein (CETP).

We have now shown that obicetrapib crosses the blood-brain barrier, with measurable CNS accumulation following oral administration. Using a transgenic mouse model of Alzheimer's disease that has been further engineered to express human CETP, we demonstrate that administration of obicetrapib prevents loss of cognitive function and reduces concentrations of Aβ fragments in the soluble fraction of the brain.

### 5.4. Methods of treatment

Accordingly, in a first aspect, methods are provided for slowing progression of neurodegenerative disease in a subject who has or is at risk of developing a neurodegenerative disease. The methods comprise administering a therapeutically effective amount of obicetrapib or a pharmaceutically acceptable salt thereof to the subject.

### 5.4.1. Obicetrapib

Obicetrapib, formerly known as TA-8995, is a compound of formula (1): 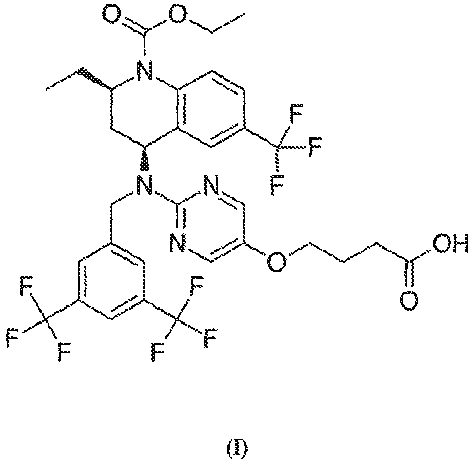 (2R,4S){[3,5Bis(trifluoromethyl)benzyl]-[5(3-carboxypropoxy)pyrimidin-2-yl] amino}-2-ethyl-6-trifluoromethyl-3,4-dihydro-2H-quinoline-1-carboxylic acid ethyl ester

Methods of synthesizing obicetrapib are known. *See, e.g.,* U.S. Patent Nos. 7,872,126; 8,084,611; and 10,112,904, the disclosures of which are incorporated herein by reference in their entireties.

### 5.4.2. Neurodegenerative diseases

In some embodiments, the subject has been diagnosed with or is at risk for Alzheimer's Disease (AD), Lewy Body dementia, vascular or multi-infarct dementia, or frontotemporal dementia (FID).

In certain embodiments, the subject has been diagnosed with or is at risk for developing AD.

In certain embodiments, the subject has been diagnosed with AD. In specific embodiments, the subject has been diagnosed with AD based on the NIA-AA. Research Framework criteria, with biomarker classification of A⁺T⁺N⁺ or A⁺T⁺N⁻ based upon (i) CSF profile consistent with AD (an Aβ42 concentration of <1000 pg/mL AND p-tau > 19 pg/mL, and/or (ii) a ratio of p-tau/Aβ42 of ≥0.024). In specific embodiments, the biomarkers are measured by Elecsys assay.

In certain embodiments, the subject has been diagnosed with AD based on documented amyloid positron emission tomography (PET) scan evidence. In certain embodiments, the subject has AD Clinical Stage 3 or 4. In specific embodiments, the subject has AD clinical stage 3 or 4 based on the NIA-AA Research Framework criteria: (i) a mini-mental state examination (MMSE) score >20 inclusive, and/or Clinical Dementia Rating scale-Sum of Boxes (CDR-SB) global score ≥0.5 and ≤1 with memory box score ≥1.0.

In certain embodiments, the subject is at risk for developing AD. In particular embodiments, the subject has at least one ApoE4 allele. In particular embodiments, the subject has two ApoE4 alleles. In some embodiments, the method further comprises the prior step of detecting the presence of ApoE4 alleles in the subject.

In some embodiments, the subject has been diagnosed with mild cognitive impairment (MCI). In certain embodiments, the diagnosis of MCI is made using the Short Test of Mental Status, the Montreal Cognitive Assessment (MoCA) or the Mini-Mental State Examination (MMSE).

In various embodiments, the subject has been determined prior to treatment to have at least one of (i) decreased levels of Aβ42 in plasma, (ii) increased levels of Aβ40 in plasma, (iii) decreased ratio of Aβ42/Aβ40 in plasma, (iv) increased levels of neurofilament light (NFL) in plasma, and (v) increased levels of neurogranin in plasma, as compared to a healthy control population that does not have neurodegenerative disease.

In various embodiments, the subject has been determined prior to treatment to have at least one of (i) decreased levels of Aβ42 in CSF, (ii) increased levels of Aβ40 in CSF, (iii) decreased ratio of Aβ42/Aβ40 in CSF, (iv) increased levels of neurofilament light (NFL) in CSF, and (v) increased levels of neurogranin in CSF, as compared to a healthy control population that does not have and that is not at elevated risk for neurodegenerative disease.

In various embodiments, the subject has been determined prior to treatment to have abnormal patterns of Tau based on Tau PET imaging.

### 5.4.3. Dose regimen

In various embodiments, obicetrapib or salt thereof is administered orally. In typical embodiments, obicetrapib is administered as a tablet for oral administration. In various embodiments, the dose of obicetrapib or salt thereof is 2.5-25 mg by mouth per day (2.5-25 mg po QD). In some embodiments, the dose of obicetrapib or salt thereof is 5-20 mg by mouth per day (5-20 mg po QD). In some embodiments, the dose of obicetrapib or salt thereof is 10-20 mg by mouth per day (10-20 mg po QD). In specific embodiments, the daily dose is 2.5 mg po QD, 5 mg po QD, 10 mg po QD, 15 mg po QD, 20 mg po QD or 25 mg po QD. In various embodiments, the dose is administered once per day. In some embodiments, the dose is divided and the 2.5-25 mg total daily dose, or 5-20 mg total daily dose, or 10-20 mg total daily dose, is administered as a plurality of divided doses.

In various embodiments, obicetrapib is administered as a tablet. In some embodiments, the tablet comprises 2.5 mg, 5 mg, 7.5 mg, 10 mg, 15 mg, 20 mg or 25 mg of obicetrapib or pharmaceutically acceptable salt thereof. In some embodiments, the tablet contains obicetrapib as the calcium salt. In particular embodiments, the table contains 5 mg obicetrapib as a calcium salt.

In specific embodiments, tablets are round, 6 nun in diameter, white film-coated tablets, containing 5 mg of obicetrapib as the calcium salt. In specific embodiments, tablets are round, 6 mm in diameter, white film-coated tablets, containing 10 mg of obicetrapib as the calcium salt. In specific embodiments, the excipients present in the tablet cores are microcrystalline cellulose, mannitol, sodium starch glycollate, colloidal silicon dioxide, and magnesium stearate. In specific embodiments, a commercially available film-coating formula (Opadry II white, ex Colorcon) is applied to the cores.

In various embodiments, obicetrapib or salt thereof is administered once daily for at least 3 weeks, at least 6 months, at least 12 months, at least 24 months, or at least 36 months.

### 5.5. Further embodiments

### 5.5.1. Dose regimen

In various embodiments, obicetrapib or pharmaceutically acceptable salt thereof is administered in an amount effective to increase levels of total ApoE-HDL in blood as compared to the level prior to commencement of treatment. In typical embodiments, blood levels of ApoE-HDL are measured in plasma. In preferred embodiments, obicetrapib or salt thereof is administered in an amount effective to increase plasma levels of ApoE-HDL in particles lacking ApoC3 and/or percentage of plasma HDL-ApoE in HDL particles lacking ApoC3.

### 5.5.2. Clinical endpoints

In various embodiments, the dose of obicetrapib or salt thereof is sufficient to prevent at least one of (i) further decrease in levels of Aβ42 in plasma, (ii) further increase in levels of Aβ40 in plasma, (iii) further decrease in ratio of Aβ42/Aβ40 in plasma, (iv) further increase in levels of neurofilament light (NFL) in plasma, and (v) further increase in levels of neurogranin in plasma, as compared to levels prior to start of treatment.

In various embodiments, the dose of obicetrapib or salt thereof is sufficient to prevent at least one of (i) further decrease in levels of Aβ42 in CSF, (ii) further increase in levels of Aβ40 in CSF, (iii) further decrease in ratio of Aβ42/Aβ40 in CSF, (iv) further increase in levels of neurofilament light (NFL) in CSF, and (v) further increase in levels of neurogranin in CSF, as compared to levels immediately prior to start of treatment.

### 5.5.3. Further numbered embodiments

1. A method of slowing progression of neurodegenerative disease in a subject who has or is at risk of developing a neurodegenerative disease, comprising:
   administering a therapeutically effective amount of obicetrapib or a pharmaceutically acceptable salt thereof to the subject.
2. The method of embodiment 1, wherein the amount is effective to increase levels of total ApoE HDL in blood.
3. The method of embodiment 1 or embodiment 2, wherein the amount is effective to increase levels of ApoE-HDL in blood.
4. The method of embodiment 3, wherein the amount is effective to increase levels of ApoE-HDL in particles lacking ApoC3 in blood.
5. The method of any one of embodiments 1-4, wherein obicetrapib or salt thereof is administered orally.
6. The method of embodiment 5, wherein obicetrapib is administered once per day.
7. The method of embodiment 6. wherein the dose is 5-20 mg po QD of obicetrapib or salt thereof.
8. The method of embodiment 7, wherein the dose is 5 mg po QD.
9. The method of embodiment 7, wherein the dose is 10 mg po QD.
10. The method of embodiment 7, wherein the dose is 15 mg po QD.
11. The method of embodiment 7, wherein the dose is 20 mg po QD.
12. The method of any one of embodiments 7-11, wherein obicetrapib or salt thereof is administered once daily for at least 8 weeks.
13. The method of embodiment 12, wherein obicetrapib or salt thereof is administered once daily for at least 6 months.
14. The method of embodiment 13, wherein obicetrapib or salt thereof is administered once daily for at least 12 months.
15. The method of embodiment 14, wherein obicetrapib or salt thereof is administered once daily for at least 24 months.
16. The method of any one of embodiments 1-15, wherein the subject has been diagnosed with or is at risk for Alzheimer's Disease (AD), Lewy Body dementia, vascular or multi-infarct dementia, or frontotemporal dementia (FTD).
17. The method of embodiment 16, wherein the subject has been diagnosed with or is at risk for developing AD.
18. The method of embodiment 17, wherein the subject has been diagnosed with AD.
19. The method of embodiment 17 or embodiment 18, wherein the subject has at least one ApoE4 allele.
20. The method of embodiment 19, wherein the subject has two ApoE4 alleles.
21. The method of any one of embodiments 19-20, further comprising the prior step of detecting ApoE4 alleles in the subject.
22. The method of any one of embodiments 1-21, wherein the subject has been diagnosed with mild cognitive impairment (MCI).
23. The method of any one of embodiments 1-22, wherein the subject has been determined prior to treatment to have at least one of (i) decreased levels of Aβ42 in plasma, (ii) increased levels of Aβ40 in plasma, (iii) decreased ratio of Aβ42/Aβ40 in plasma, (iv) increased levels of neurofilament light (NFL) in plasma, and (v) increased levels of neurogranin in plasma, as compared to a healthy control population that does not have and that is not at elevated risk for neurodegenerative disease.
24. The method of any one of embodiments 1-23, wherein the subject has been determined prior to treatment to have at least one of (i) decreased levels of Aβ42 in CSF, (ii) increased levels of Aβ40 in CSF, (iii) decreased ratio of Aβ42/Aβ40 in CSF, (iv) increased levels of neurofilament light (NFL) in CSF, and (v) increased levels ofneurogranin in CSF, as compared to a healthy control population that does not have and that is not at elevated risk for neurodegenerative disease.
25. The method of any one of embodiments 1-24, wherein the subject has been determined prior to treatment to have abnormal patterns of Tau based on Tau PET imaging.
26. The method of any one of embodiments 1-25, wherein the dose of obicetrapib or salt thereof is sufficient to prevent at least one of (i) further decrease in levels of Aβ42 in plasma, (ii) further increase in levels of Aβ40 in plasma, (iii) further decrease in ratio of Aβ42/Aβ40 in plasma, (iv) further increase in levels of neurofilament light (NFL) in plasma, and (v) further increase in levels of neurogranin in plasma, as compared to levels immediately prior to start of treatment.
27. The method of any one of embodiments 1-26, wherein the dose of obicetrapib or salt thereof is sufficient to prevent at least one of (i) further decrease in levels of Aβ42 in CSF, (ii) further increase in levels of Aβ40 in CSF, (iii) further decrease in ratio of Aβ42/Aβ40 in CSF, (iv) further increase in levels of neurofilament light (NFL) in CSF, and (v) further increase in levels of neurogranin in CSF, as compared to levels immediately prior to start of treatment.
28. The method of any one of embodiments 1-27, wherein obicetrapib is administered as a tablet.
9. The method of embodiment 28, wherein the tablet comprises obicetrapib as the calcium salt.
30. The method of embodiment 28 or 29, wherein the tablet comprises one or more excipients.
31. The tablet of embodiment 30 wherein the one or more excipients comprises microcrystalline cellulose, mannitol, sodium starch glycollate, colloidal silicon dioxide, and magnesium stearate or combinations thereof.

### 6. EXAMPLES

### 6.1. Example 1: Qualitative Whole Body Analysis Demonstrates That Obicetrapib Crosses The Blood-Brain Barrier

To determine the tissue distribution of obicetrapib and/or its metabolites post-dose, and particularly to determine distribution into brain tissues, we employed the use of radiolabeled [¹⁴C]-obicetrapib and quantitative whole-body techniques.

The mice were maintained in rooms at a temperature of 19 to 25°C, with a relative humidity of between 40 and 70%, and exposed to fluorescent light (nominal 12 hours) each day. They were permitted free access to a Western-type diet (semi-synthetic modified diet containing 15% cacao butter, 1% corn oil and 40.5 sucrose). Equal numbers of male (n = 5) and female mice (n = 5) were given a single administration by gavage at 5g/kg of [¹⁴C]-obicetrapib. The mice were humanely sacrificed at 1 hour, 4 hours, 24 hours, 72 hours and 168 hours from time of dosing, following which blood and tissue samples were retrieved.

Using blood plasma as the reference point, the data (see Tables 1-4 below) indicate that in female mice 1 hour post-dose, the concentration of [¹⁴C]-obicetrapib in tissues of the brain is about 4% the concentration in plasma. At the 4 hour mark, that relative concentration rises significantly to about 16% of the concentration in plasma. Specifically, in the brain choroid plexus, the radiolabeled drug product is found in concentrations almost 50% of blood plasma concentrations. Also notable is an observed relative concentration of almost 17% in the cerebellum. Compared with the female mice, the male counterparts displayed similar overall concentrations in the brain, while showing slightly lower concentration in the cerebellum. These data indicate that at 4 hours after an oral dose of 5g/kg there is significant obicetrapib distribution into the brain.

| **Table 1** | | | | | |
|---|---|---|---|---|---|
| **Concentration Following Oral Dose Of [¹⁴C]-Obicetrapib In Female Mice (Concentration Units ngEq/g)** | | | | | |
| | **Female** | **Female** | **Female** | **Female** | **Female** |
| Sample | 1 h | 4 h | 24 h | 72 h | 168 h |
| Blood (QWBA) | 3890 | 6940 | 7710 | 1790 | BLQ |
| Plasma (LSC) | 8520 | 13100 | 13800 | 3250 | 182 |
| Brain | 380 | 1910 | 1040 | BLQ | BLQ |
| Brain cerebellum | 422 | 2210 | 1090 | BLQ | BLQ |
| Brain cerebrum | 456 | 2010 | 1010 | BLQ | BLQ |
| Brain choroid plexus | 1910 | 5600 | 1590 | BLQ | BLQ |
| Brain medulla | 384 | 1780 | 1130 | BLQ | BLQ |
| Brain olfactory lobe | 356 | 1980 | 908 | BLQ | BLQ |
| Brain thalamus | 406 | 2090 | 996 | BLQ | BLQ |

| **Table 2** | | | | | |
|---|---|---|---|---|---|
| **Tissue:Plasma Concentration Ratios Following Oral Dose Of [¹⁴C]-Obicetrapib In Female Mice** | | | | | |
| | **Female** | **Female** | **Female** | **Female** | **Female** |
| Sample | 1 h | 4 h | 24 h | 72 h | 168 h |
| Blood (QWBA) | 0.456 | 0.529 | 0.559 | 0.553 | NC |
| Plasma (LSC) | 1.00 | 1.00 | 1.00 | 1.00 | 1.00 |
| Brain | 0.0446 | 0.146 | 0.0754 | NC | NC |
| Brain cerebellum | 0.0495 | 0.168 | 0.0789 | NC | NC |
| Brain cerebrum | 0.0536 | 0.153 | 0.0730 | NC | NC |
| Brain choroid plexus | 0.224 | 0.427 | 0.116 | NC | NC |
| Brain medulla | 0.0451 | 0.136 | 0.0819 | NC | NC |
| Brain olfactory lobe | 0.0417 | 0.151 | 0.0659 | NC | NC |
| Brain thalamus | 0.0477 | 0.159 | 0.0723 | NC | NC |

| **Table 3** | | | | | |
|---|---|---|---|---|---|
| **Concentration Following Oral Dose Of [¹⁴C]-Obicetrapib In Mice (Concentration Units ngEq./g)** | | | | | |
| | **Male** | **Male** | **Male** | **Male** | **Male** |
| Sample | 1 h | 4 h | 24 h | 72 h | 168 h |
| Blood (QWBA) | 3130 | 5710 | 6100 | 2420 | BLQ |
| Plasma (LSC) | 5530 | 12700 | 11600 | 4490 | 395 |
| Brain | 331 | 1640 | 1020 | BLQ | BLQ |
| Brain cerebellum | 381 | 1820 | 1060 | BLQ | BLQ |
| Brain cerebrum | 336 | 1720 | 1000 | BLQ | BLQ |
| Brain choroid plexus | 1380 | 8040 | 2440 | 490 | BLQ |
| Brain medulla | 333 | 1620 | 1050 | BLQ | BLQ |
| Brain olfactory lobe | BLQ | 1570 | 894 | BLQ | BLQ |
| Brain thalamus | 380 | 1650 | 1020 | BLQ | BLQ |

| **Table 4** | | | | | |
|---|---|---|---|---|---|
| **Tissue:plasma concentration ratios following oral dose of [14C]-Obicetrapib in Mouse** | | | | | |
| | **Male** | **Male** | **Male** | **Male** | **Male** |
| Sample | 1 h | 4 h | 24 h | 72 h | 168 h |
| Blood (QWBA) | 0.566 | 0.449 | 0.524 | 0.539 | NC |
| Plasma (LSC) | 1.00 | 1.00 | 1.00 | 1.00 | 1.00 |
| Brain | 0.0598 | 0.129 | 0.0877 | NC | NC |
| Brain cerebellum | 0.0690 | 0.143 | 0.0907 | NC | NC |
| Brain cerebrum | 0.0609 | 0.135 | 0.0861 | NC | NC |
| Brain choroid plexus | 0.249 | 0.632 | 0.209 | 0.109 | NC |
| Brain medulla | 0.0602 | 0.127 | 0.0901 | NC | NC |
| Brain olfactory lobe | NC | 0.123 | 0.0768 | NC | NC |
| Brain thalamus | 0.0687 | 0.130 | 0.0875 | NC | NC |

### 6.2. Example 2: Preclinical Assessment Of The CETP Inhibitor Obicetrapib For The Prevention Of Alzheimer's Disease (AD)

### 6.2.1. Study Methodology

To assess the potential efficacy of obicetrapib to slow onset and/or progression of Alzheimer's disease (AD), we performed the following preclinical mouse experiments.

Mice do not express CETP. Mouse strains used for this trial thus include a CETP transgenic mouse strain expressing human CETP under control of the human endogenous promoter (B6.CBA-Tg(CETP)5203Tall/J, Jackson Labs). The promoter is sensitive towards dietary cholesterol intake, so that these mice, when fed with a 1% (w/w) high cholesterol diet, show increased CETP expression.

Also used in this trial is the APP tg mouse strain McGill-Thyl -APP-Cuello-Tg. The McGill-Thy1-APP transgenic rat expresses human APP751 with the Swedish and Indiana mutations, under the control of the murine Thy1.2 promoter. These rats are among the most extensively studied of the existing APP transgenic rat lines. Homozygous animals show age-dependent accumulation of amyloid plaques, gliosis, cholinergic synapse loss, and cognitive impairment. See Petrasek et al., Front Aging Neurosci., vol. 10, Article 250 (2018).

Transgenes were maintained heterozygously for each line. For the generation of mice entering the trial, CETP tg females or males were bred with APP tg males or females, respectively. The offspring included wild type, CETP tg only, APP tg only, and hCETP/APP tg genotypes, so that all mice entering the trial are littermates (Figure 1). Food and drinking water was provided ad libitum with a 12 h day/night light cycle.

Mice were bred and maintained on a standard diet. At 12 weeks of age, mice were put on a 1% (w/w) cholesterol diet (Research Diets) to induce CETP expression, and in parallel either received obicetrapib (dose 30 mg/kg) or vehicle control via intraperitoneal injection. Obicetrapib was dissolved in a mixture of 30% Kolliphor (#C5135 Millipore Sigma) and 60% isotonic glucose solution undergoing repeating cycles of warming the solution and dissolving the powder in the ultrasonic water bath. The trial ended when mice reached 24 weeks of age, so that the total exposure of mice to cholesterol diet and treatment summed up to 12 weeks (Figure 1). Mice were then sacrificed, and various tissue extracts were collected.

| **Table 5** | | | |
|---|---|---|---|
| **Disposition of animals** | | | |
| | **obicetranib** | **vehicle** | **total mice** |
| **WT** | 10 males, 10 females | 10 males, 10 females | 40 |
| **CETP tg** | 10 males, 10 females | 10 males, 10 females | 40 |
| **APP tg** | 10 males, 10 females | 10 males, 10 females | 40 |
| **hCETP/APP dtg** | 10 males, 10 females | 10 males, 10 females | 40 |
| **SUM** | | | 160 mice |

| | | | |
|---|---|---|---|
| WT == wildtype; CETP = cholesteryl ester transfer protein; tg = transgenic; APP = amyloid precursor protein; dtg = double transgenic. | | | |

To confirm that the drug reached its target, blood was drawn from mice at 4 hours and 24 hours after intraperitoneal injection. Following that, CETP activity was measured using the Roar Biomedical Inc. fluorescent CETP activity assay (product no. RB-CETP) as follows: one microliter of mouse plasma at a 1:15 dilution, was incubated with 0.3 µL donor and 0.3 µL acceptor particles in 30 µL reaction buffer. Fluorescence was measured following an incubation period of 3 hours at 37°C in a water bath. The results indicate that obicetrapib effectively inhibited human CETP in mouse plasma 4 hours as well as 24 hours post-dose (Figure 2).

### 6.2.2. Obicetrapib Reduces Deterioration Of Cognitive Performance In hCETP/APP Double Transgenic Mice

Nest building is a natural behavior of mice to preserve energy, hide and mate. Cognitively intact mice build competent nests. This nesting phenomenon has been shown to be sensitive towards hippocampal disruptions; inability to nest is indicative of cognitive decline.

During the final week of the trial period (week 23-24), each mouse was placed in a clean cage for 10-12 hours with 2.0-2.5 grams of bedding material, and food and water *ad libitum.* The following morning the quality of the nests were assessed on a 5-point scale. The data show that wild-type mice on vehicle, wild-type mice on obicetrapib, CETP mice on vehicle, and CETP mice on obicetrapib show approximately equal nesting behavior. In contrast, APP mice on vehicle, APP mice on obicetrapib, and hCETP/APP double transgenic mouse on vehicle show great disruption in their ability to build nests. However, hCETP/APP double transgenic mice dosed with obicetrapib retain the ability to construct nests, indicating that obicetrapib reduces cognitive decline in the hCETP/APP double transgenic mice (Figure 3A-B).

### 6.2.3. Obicetrapib Reduces Soluble Amyloid-B Fragments In Brain Of Heetp/APP Double Transgenic Mice. Quantification By ELISA

Following our cognitive analysis experiments, mice were sacrificed and brain samples were collected. The Aβ load was quantified from the brain extracts. Aβ load was measured using the V-PLEX Plus Aβ Peptide Panel 1 (6E10) Kit (Mesoscale discovery) according to manufacturer's instructions. Briefly, 50 mg of cortex tissue on average was mechanically homogenized a 5x volume of cold tissue homogenization buffer (2 mM Tris, pH 7.4, 250 mM Sucrose, 0.5 mM EDTA, 0.5 mM EGTA, 1x protease inhibitor cocktail). The resulting homogenate was centrifuged in a table-top centrifuge at 5000 x g for 10 min at 4°C. A total of 180 µl was taken from the resulting supernatant and mixed with 180 µl DEA buffer (0.4% DEA, 0.1 M NaCl). From the mixture, 200 µl were transferred to fresh tubes and centrifuged at 120000 x g for 1 h at 4 °C. 150 µL supernatant were neutralized with 16 µl 0.5 M Tris-HCl, pH 6.8 buffer, resulting in the soluble DEA extract. The pellet was dissociated in 125 µL cold 70% formic acid (FA) for 30 min on ice, following centrifugation at 120000 x g for 1 h at 4 °C. 105 µL supernatant was neutralized with 1400 µl neutralization buffer (1 M Tris base, 0.5 M Na₃HPO₄, 0.05% NaN₃) resulting in the FA-soluble extract. 25 µL of soluble DEA extract and FA-extracts were used in the ELISA.

In Figure 4, the APP single transgenic mice and hCETP/APP double transgenic mice displayed a higher Aβ load versus wild-type. The data further demonstrate that obicetrapib decreases Aβ load in both transgenic mice models with higher decreases observed in the hCETP/APP double transgenic mice.

### 6.3. Example 3: A Placebo-Controlled, Double-Blind, Randomized, Phase 2 Dose-Finding Study to Confirm the Effect of Obicetrapib as an Adjunct to High-Intensity Statin Therapy

### 6.3.1. Study Design Summary

A randomized, placebo-controlled, double-blind, Phase 2 dose-finding study (ROSE Trial, NCT04753606) was conducted to confirm the efficacy, safety, and tolerability of obicetrapib as an adjunct to high-intensity statin therapy.

### 6.3.2. Specific Objectives

The primary objective of this study was to confirm the efficacy of obicetrapib, compared to placebo, at Day 56 in decreasing low-density lipoprotein cholesterol (LDL-C) as an adjunct to high-intensity statin therapy.

### 6.3.3. Treatment Period

Up to 2 weeks after the Screening Visit, participants returned to the site on Day 1 (Visit 2) and confirmed study eligibility before being randomized and beginning treatment. Approximately 114 eligible participants (38 participants per treatment group) were randomized in a 1:1:1 ratio to one of the following treatment groups:
- 5 mg obicetrapib (one 5 mg obicetrapib tablet + 1 placebo tablet);
- 10 mg obicetrapib (two 5 mg obicetrapib tablets); or
- Placebo (2 placebo tablets).

During the 8-week Treatment Period, the assigned study drugs were administered to the participants orally, once daily, on Day 1 to Day 56. Participants returned to the site every 4 weeks for efficacy, safety, and pharmacokinetic (PK) assessments. Participants, Investigators, the Clinical Research Organization (CRO), and the Sponsor were blinded to all lipid results from Day 1 (Visit 2) for the first participant through database lock in order to protect blinding to treatment assignment.

### 6.3.4. Safety Follow-Up Period

Participants returned to the site for a Safety Follow-up Visit (Visit 5) approximately 4 weeks after the end of the Treatment Period for safety and PK assessments.

### 6.3.5. Pharmacokinetic Period

Participants returned to the site for two PK Visits (Visits 6 and 7) approximately 8 and 15 weeks, respectively, after the end of the Treatment Period for safety and PK assessments.

### 6.3.6. Inclusion Criteria

Participants who met all of the following criteria were eligible to participate in the study:
1. Understanding of the study procedures, willingness to adhere to the study schedules, and agreement to participate in the study by giving written informed consent prior to screening procedures;
2. Men or women 18 to 75 years of age, inclusive, at the Screening Visit;
   - Women may be enrolled if all 3 of the following criteria were met:
      i. They were not pregnant;
      ii. They were not breastfeeding; and
      iii. They did plan on becoming pregnant during the study;
   - Women of childbearing potential must have had a negative urine pregnancy test at the Screening Visit. Note: Women were not considered to be of childbearing potential if they met 1 of the following criteria as documented by the Investigator:
      i. They had a hysterectomy or tubal ligation at a minimum of 1 cycle prior to signingthe ICF; or
      ii. They were postmenopausal, defined as ≥1 year since their last menstrual period for women ≥55 years of age or ≥1 year since their last menstrual period and had a follicle-stimulating hormone (FSH) level in the postmenopausal range for women <55 years of age; and
   - Women of childbearing potential must have agreed to use an effective method of avoiding pregnancy from screening to 90 days after the last visit. Men whose partners are of childbearing potential must have agreed to use an effective method of avoiding pregnancy from screening to 90 days after the last visit. Effective methods of avoiding pregnancy are contraceptive methods with a Pearl index of <1 used consistently and correctly (including implantable contraceptives, injectable contraceptives, oral contraceptives, transdermal contraceptives, intrauterine devices, diaphragm with spermicide, male or female condomswith spermicide, or cervical cap) or a sterile sexual partner;
3. Fasting LDL-C levels >70 mg/dL and TG levels <400 mg/dL at the Screening Visit; and
4. Currently receiving high-intensity statin therapy (atorvastatin 40 mg or 80 mg per day or rosuvastatin 20 or 40 mg per day) at a stable dose for 8 weeks prior to screening and intended to remain at the same stable dose throughout the study duration.

### 6.3.7. Exclusion Criteria

Participants who met any of the following criteria were excluded from participation in the study:
1. Body mass index ≥40 kg/m2 at the Screening Visit;
2. Current clinically significant CV disease, including but not limited to:
   a. Major adverse CV event within 3 months prior to randomization; or
   b. New York Heart Association Functional Classification Class III or IV heart failure;
3. Glycosylated hemoglobin (HbA1c) ≥10% at the Screening Visit;
4. Uncontrolled hypertension, i.e., sitting systolic blood pressure >160 mmHg and/or sitting diastolic blood pressure >90 mmHg. One retest was allowed, at which point if the retest result was no longer exclusionary, the participant might be randomized;
   Active muscle disease or persistent creatine kinase concentration >3 × the upper limit of normal (ULN). One retest was allowed after 1 week to verify the result, at which point if the retest result was no longer exclusionary, the participant might be randomized;
   Estimated glomerular filtration rate <60 mL/min, calculated using the Chronic Kidney Disease Epidemiology Collaboration equation;
   Hepatic dysfunction as evidenced by any laboratory abnormality as follows: gamma-glutamyl transferase, alanine aminotransferase, or aspartate aminotransferase >2 × ULN, or total bilirubin >1.5 × ULN;
   Anemia, defined as hemoglobin concentration <11 g/dL for men and hemoglobin concentration <9 g/dL for women;
   History of malignancy within 5 years prior to screening, with the exception of non-melanoma skin cancers;
   History of alcohol and/or drug abuse within 5 years prior to screening;
   Treatment with other investigational products or devices within 30 days or 5 half-lives, whichever was longer, prior to screening;
   Treatment with any proprotein convertase subtilisin kexin type 9 (PCSK9) inhibitor within 10 weeks prior to randomization or bempedoic acid within 2 weeks prior to randomization;
   Evidence of any other clinically significant, non-cardiac disease or condition that, in the opinion of the Investigator, would have precluded participant in the study; or
   Known CETP inhibitor allergy or intolerance.

### 6.3.8. Retesting

If laboratory abnormalities during screening were considered by the Investigator to be transient, then the laboratory tests may have been repeated once during screening. The Investigator's rationale for retesting was documented. If the retest result was no longer exclusionary, the participant may have been randomized.

### 6.3.9. Rescreening

A participant who was screened and did not meet the study eligibility criteria was considered for rescreening upon Sponsor and/or Medical Monitor consultation and approval. Rescreened participants were assigned a new participant number. Rescreening occurred no less than 5 days after the last Screening Visit.

### 6.3.10. Withdrawal Criteria

Participation in this study may have been discontinued for any of the following reasons:
1. The participant withdrew consent or requested discontinuation from the study for any reason;
2. Occurrence of any medical condition or circumstance that exposed the participant to substantialrisk and/or did not allow the participant to adhere to the requirements of the protocol;
3. Any SAE, clinically significant AE, severe laboratory abnormality, intercurrent illness, or other medical condition, which indicated to the Investigator that continued participation was not in the best interest of the participant;
4. Pregnancy;
5. Requirement of prohibited concomitant medication;
6. Participant failed to comply with protocol requirements or study-related procedures; or
7. Termination of the study by the Sponsor or the regulatory authority.

Unless the participant withdrew consent, participants who discontinued study drug early were encouraged to complete the full panel of assessments scheduled for the Early Termination Visit promptly and complete a Safety Follow-up Visit 4 weeks after the last dose. If a participant withdrew prematurely from the study due to the above criteria or any other reason, they were to make every effort to complete the full panel of assessments scheduled for the Early Termination Visit and complete a Safety Follow-up Visit 4 weeks after the last dose. PK samples were not to be collected during the Safety Follow-up Visit for participants who discontinued study drug early without widtdrawing consent or for participants who withdrew prematurely from the study. The reason for participant withdrawal was documented in the electronic case report form (eCRF).

In the case of participant lost to follow-up, attempts to contact the participant were to be made and documented in the participant's medical records. Withdrawn participants were not replaced.

### 6.3.11. Study Treatments

### 6.3.11.1 Treatment Groups

Participants were randomized in a 1:1:1 ratio to one of the following treatment groups:
1. 5 mg obicetrapib (one 5 mg obicetrapib tablet + 1 placebo tablet);
2. 10 mg obicetrapib (two 5 mg obicetrapib tablets); or
3. Placebo (2 placebo tablets).

### 6.3.11.2 Rationale for Dosing

In previous clinical studies of obicetrapib in healthy volunteers and patients, near maximal effectswere observed at the 5 mg obicetrapib dose level with increased HDL-C levels and decreased LDL-C levels. A statistically significant reduction in Lp(a) levels from baseline was also observed at the 10 mg obicetrapib dose level. Therefore, the present study utilized doses of 5 and 10 mg obicetrapib in participants currently receiving high-intensity statin therapy.

### 6.3.11.3 Randomization and Blinding

Participants who met all eligibility criteria were randomized into the study. Participants were randomized in a 1:1:1 ratio to the (i) 5 mg obicetrapib, (ii) 10 mg obicetrapib, or (iii) placebo treatment groups. At randomization, participants were stratified according to their Screening Visit (Visit 1) LDL-C level (≥1.00 or <100 mg/dL). An automated interactive response technology (IRT) system was used to assign the participant to 1 of the 3 treatment groups.

Participants, Investigators, the CRO, and the Sponsor were blinded to all lipid results from Day 1(Visit 2) for the first participant through database lock in order to protect blinding to treatment assignment.

### 6.3.12. Drug Supplies

### 6.3.12.1 Formulation and Packaging

The study drugs consisted of 5 mg obicetrapib tablets and matching placebo tablets. All study drugs are manufactured in accordance with current Good Manufacturing Practice.

Obicetrapib tablets are round, 6 mm in diameter, white film-coated tablets, with no identifying markings, containing 5 mg of obicetrapib drug substance. Matching placebo tablets were also provided. The excipients present in the tablet cores are microcrystalline cellulose, mannitol, sodium starch glycollate, colloidal silicon dioxide, and magnesium stearate. A commercially available film-coating formula (Opadry II white, ex Colorcon) is applied to the cores.

Obicetrapib and placebo tablets were packaged into foil blisters and assembled into blister cards providing the study drugs for each treatment group. The blister cards were clearly labelled to indicate which blisters to use on each day. The tablets were stored at ambient conditions below 25 °C (77 °F).

The physical, chemical, and pharmaceutical formulation properties and characteristics of the obicetrapib tablets were described in the Investigator's Brochure.

All study drugs were labelled in accordance with all applicable local regulatory requirements.

### 6.3.12.2 Study Drug Preparation and Dispensing

The study drugs used in this study are as follows:
1. 5 mg obicetrapib tablet; and
2. Matching placebo tablet.

The study drugs listed above were packaged to provide doses of 5 or 10 mg obicetrapib or placebo only. Participants were randomized to receive 1 of the 2 doses of obicetrapib or placebo only on Day 1 to Day 56.

At each appropriate visit (Visits 2 and 3), participants received 4 blister cards with the study drugs appropriate for the participant's treatment group. Each blister card provided a sufficient supply for 1 week of dosing, with enough for an extra day of dosing in case the participant needed to postpone the next visit. Participants were instructed to take 2 units from the blister cards each day. The blister cards were clearly labelled to indicate which blisters to use on each day. Participants were instructed to bring all unused study drugs to the site at the next visit.

### 6.3.13. Study Drug Administration

Two tablets of study drugs were self-administered by the participant orally, once daily, on Day 1 to Day 56. Study drugs were administered at approximately the same time each morning, with food On days with visits scheduled, study drugs were administered with food following all fasted blood samples. If a participant forgot to take study drug on a given day, they were to take the next dose as normal and should not take a double dose to make up for the forgotten dose.

### 6.3.14. Treatment Compliance

Compliance to the study drug regimen is evaluated by counting unused tablets. Participants were instructed to bring all unused study drugs to the site at Visits 3 and 4. During the Treatment Period, if compliance was not between 80% and 120%, inclusive, the participant was counselled about the importance of compliance to the regimen. If the limits were exceeded at 2 consecutive visits, a decision was made by the Investigator and Sponsor as to whether the participant should be withdrawn from the study.

### 6.3.15. Storage and Accountability

All study drugs were stored below 25 °C (77 °F) in a secure area with access limited to the Investigator and authorized site personnel.

In accordance with regulatory requirements, the Investigator or designated site personnel documented the amount of study drug dispensed and/or administered to participants, the amount returned by participants, and the amount received from and returned to the Sponsor (or representative) when applicable. Study drug accountability records were maintained throughout the course of the study. The accountability unit for this study was a tablet. Discrepancies were to be reconciled or resolved. Procedures for final disposition of unused study drug were provided in the appropriate study manual.

### 6.3.16. Prior and Concomitant Medications and/or Procedures

### 6.3.16.1 Excluded Medications and/or Procedures

Participants were not to receive treatment with other investigational products or devices within 30 days or 5 half-lives, whichever was longer, prior to screening.

Participants were to abstain from taking any PCSK9 inhibitor within 10 weeks prior to randomization or bempedoic acid within 2 weeks prior to randomization.

### 6.3.16.2 Allowed Medications and/or Procedures

Participants were to be currently receiving high-intensity statin therapy (atorvastatin 40 or 80 mg per day or rosuvastatin 20 or 40 mg per day) at a stable dose for 8 weeks prior to screening and intending to remain at the same stable dose throughout the study duration.

### 6.3.16.3 Documentation of Prior and Concomitant Medication Use

Medications used within 28 days prior to the Screening Visit were recorded. Any medications administered in addition to the study drugs, whether allowed per the protocol or not, were to be documented on the concomitant medication eCRF.

### 6.3.17. Study Procedures

Study procedures follows the Schedule of Procedures:

| | **Screening** | **TreatmentPeriod** | | | **Safety Follow-Up** | **PK** | | **Early Termination Visit** |
|---|---|---|---|---|---|---|---|---|
| **Visit** | **1** | **2** | **3** | **4** | **5** | **6** | **7** | |
| **Week** | **Up to -2** | **0** | **4** | **8** | **12** | **1** | **2** | |
| | | | | | | **6** | **3** | |
| **Day (± Visit Window)** | **-14 to -1** | **1** | **28 (±2)** | **56 (±2)** | **84** (**±2**) | **112 (±2)** | **161 (±2)** | **Unscheduled** |
| Informed consent^{c} | X | | | | | | | |
| Inclusion/exclusion criteria | X | X^{d} | | | | | | |
| Demographic information | X | | | | | | | |
| Medical/surgical history | X | | | | | | | |
| Prior/concomitant medications | X | X | X | X | X | | | |
| Weight and height^{e} | X | | X | X | X | | | X |
| Physical examination | X | | | X | | | | |
| Vital signs^{f} | X | X | X | X | X | | | |
| 12-lead ECG^{R} | X | | | | | | | X |
| Urine pregnancy test^{h} | X | | | | | | X | X |
| FSH testⁱ | X | | | | | | | |
| Fasting (approximately 10 hours) chemistry and hematology^{j} | X | X | X | X | X | X | X | X |
| Fasting (approximately 10 hours)lipid profile^{k} | X | X | X | X | | | | X |
| PK sample^{l} | | X | X | X | X^{m} | X | X | X |
| Randomization | | X | | | | | | |
| Dispense study drug | | X | X | | | | | |
| Study drug administrationⁿ | | X | X | X | | | | |
| Study drug compliance | | | X | X | | | | |
| Register visit in IRT | X | X | X | X | | | | X |
| Adverse events | | X | X | X | X | X | X | X |

COVID-19 = Coronavirus Disease 2019; ECG = electrocardiogram; FSH = follicle-stimulating hormone; HbAlc = glycosylated hemoglobin; IRT = interactive response technology; LDL-C = low-density lipoprotein cholesterol; PK = pharmacokinetic; TG = triglycerides.

### 6.3.18. Clinical Laboratory Evaluations

Blood for chemistry and hematology was obtained as indicated in the Schedule of Procedures and sent to a central laboratory for analysis. In particular, the following lipid profile data were collected:
Apolipoprotein B
Apolipoprotein E (ApoE)
High-density lipoprotein-ApoE [1]
High-density lipoprotein cholesterol
Low-density lipoprotein cholesterol [2]
Non-high-density lipoprotein cholesterol
Triglycerides
Very low-density lipoprotein cholesterol

[note 1] With and without apolipoprotein C3.
[note 2] Calculated using the Friedewald equation unless triglycerides ≥400 mg/dL or low-density lipoprotein cholesterol (LDL-C) ≤50 mg/dL; in both cases, LDL-C level was measured directly by preparative ultracentrifugation, also referred to as beta quantification. (Source: LDL calculated. MDCalc. https://www.mdcalc.com/ldl-calculated. Accessed 09 November 2020.) In addition, for all patients, LDL-C was measured by preparative ultracentrifugation, also referred to as beta quantification, at baseline (Visit 2) and at the end of the 8-week Treatment Period (Visit 4).

Blood samples for chemistry and hematology were obtained under fasting conditions (i.e., after the participant has fasted for approximately 10 hours). For the purposes of this study, fasting was defined as nothing by mouth except water and any essential medications. If a participant was not fasting, the Investigator was to reschedule the visit as soon as possible. Estimated glomerular filtration rate is calculated using the Chronic Kidney Disease Epidemiology Collaboration equation. At the Screening Visit only, the chemistry panel includes HbA1c.

A urine pregnancy test was performed for women of childbearing potential at the Screening Visit prior to their participation in the study, the last PK Visit (Visit 7), and the Early Termination Visit.

An FSH test was performed at the Screening Visit prior to participation in the study in women <55 years of age for whom it has been ≥1 year since their last menstrual period.

Blood samples for PK assessment were collected as indicated in the Schedule of Procedures. On Day 1, a PK sample is collected pre-dose. The subsequent post-dose PK samples were collected once at approximately the same time at each visit.

### 6.3.19. Weight and Height

Weight and height were measured at the Screening Visit and used to calculate body mass index. Measurement of weight was performed with the participant dressed in indoor clothing,with shoes removed, and bladder empty.

### 6.3.20. Demographics

Participant demographic data (e.g., gender, race, ethnicity, and birth date/year) were collected at the Screening Visit.

### 6.3.21. Electrocardiograms

A single, standard 12-lead ECG was performed by the Investigator or trained site personnel at the Screening Visit and read locally.

### 6.3.22. Physical Examinations

A physical examination was performed as indicated in above Schedule of Procedures.

### 6.3.23. Efficacy Assessments

The primary efficacy endpoint is the percent change from Day 1 to Day 56 in LDL-C for each obicetrapib group compared to the placebo group.

Blood samples for the lipid profile were obtained under fasting conditions (i.e., after the participant had fasted for approximately 10 hours). For the purposes of this study, fasting was defined as nothing by mouth except water and any essential medications. If a participant was not fasting, the Investigator rescheduled the visit as soon as possible. LDL-C level was calculated using the Friedewald equation unless TG ≥400 mg/dL or LDL-C ≤50 mg/dL; in both cases, LDL-C level was measured directly by preparative ultracentrifugation, also referred to as beta quantification. In addition, for all patients, LDL-C was measured by preparative ultracentrifugation, also referred to as beta quantification, at baseline (Visit 2) and at the end of the 8-week Treatment Period (Visit 4).

### 6.3.24. Results

Recently, a large prospective case-cohort study demonstrated that in older adults, higher whole plasma apoE levels and higher apoE levels in HDL were not associated with reductions in dementia or Alzheimer's disease risk. In the same study, however, apoE levels in HDL that lacked ApoC3 were demonstrated to be associated with better cognitive function and lower dementia risk. Koch et al., "Association of apolipoprotein E in lipoprotein subspecies with risk of dementia," JAMA Network Open 3(7):e209250 (2020).

Obicetrapib, a potent CETP inhibitor, has previously been reported to increase total plasma apoE levels. Hovingh et al., "Cholesterol ester transfer protein inhibition by TA-8995 in patients with mild dyslipidaemia (TULIP): a randomized, double-blind, placebo-controlled phase 2 trial," Lancet 386:452-60 (2015;) and van Cappelleveen et al., "Effects of the cholesteryl ester transfer protein inhibitor, TA-8995, on cholesterol efflux capacity and high-density lipoprotein particle subclasses,"J. Clin. Lipidology 10:11.37-1144 (2016). The effect of obicetrapib on HDL-apoE, and apoE levels in HDL with and without ApoC3, has never been reported.

As described above, the ROSE trial was a phase 2, placebo-controlled, double-blind, randomized, clinical trial studying effects of obicetrapib as an adjunct to high-intensity statin therapy in patients who had fasting LDL-cholesterol (LDL-C) levels >70 mg/dL despite high intensity statin therapy. Results show that after 8 weeks of treatment with obicetrapib at 5 mg and 10 mg per day, total plasma HDL-ApoE levels were significantly increased (Table 6).

| **Table 6** | | | | | |
|---|---|---|---|---|---|
| **Total Plasma HDL** | | | | | |
| **Visit** | | | **Placebo (N=40)** | **Obicetrapib 5 mg (N=40)** | **Obicetrapib 10 mg (N=40)** |
| | **Treatment Paired-Wise Comparison** | | | | |
| | | **Statistic** | | | |
| Baseline | | | | | |
| | n | | 40 | 40 | 40 |
| | Mean (SD) | | 3.77 (0.870) | 3.56 (0.946) | 3.60 (1.268) |

| Day 56 | | | | | |
|---|---|---|---|---|---|
| | n | | 39 | 39 | 39 |
| | Mean (SD) | | 3.74 (0.905) | 4.64 (1.832) | 5.10 (2.172) |

| Percent change from baseline to Day 56 (%) | | | | | |
|---|---|---|---|---|---|
| | n'[1] | | 39 | 39 | 39 |
| | Mean (SD) | | 0.06 (18.335) | 32.87 (40.137) | 44.83 (38.110) |
| | LS mean (SE) [2] | | 1.25 (5.296) | 31.78 (5.272) | 44.45 (5.215) |
| | | 95%, CI | (-9.24. 11.74) | (21.34, 42.23) | (34.12. 54.7δ1 |
| | | p-value (2-sided) | 0.8145 | <0.0001 | <0.0001 |

| Comparison to placebo [2] | | | | | |
|---|---|---|---|---|---|
| | LS mean (SE) | | - | 30.54 (7.489) | 43.21 (7.439) |
| | | 95% CI | - | (15.71, 45.37) | (28.47, 57.94) |
| | | p-value (2-sided) | - | <0.0001 | <0.0001 |
| Baseline was defined as the last measurement prior to the first dose of study drug. | | | | | |
| 1. n' = number of participants with a measurement at both baseline and Day 56. | | | | | |
| 2. All participants with non-missing baseline values were included. | | | | | |
| LS means, SEs, Cis, and p-values We're from an MMRM Model with treatment, visit, and treatment by visit as factors and baseline ApoE as a continuous covariate. Missing at random was assumed for all missing data as scheduled visits. ApoE = apolipoprotein E; CI = confidence interval; LS = least squares; mITT = Modified Intent-to-Treat; MMRM = mixed model for reseated measures; SD = standard deviation; SE = standard error. | | | | | |

ApoE in plasma HDL particles lacking ApoC3 are increased, and the percentage of HDL-ApoE in particles lacking ApoC3 are increased. The increase in ApoE-HDL lacking ApoC3, and the increase in the percentage of HDL-ApoE in particles lacking ApoC3, demonstrate that obicetrapib will be effective to slow progression of neurodegenerative diseases in patients who have, or are at risk for, neurodegenerative diseases, such as Alzheimer's disease, particularly in subjects with at least one ApoE4 allele.

### 6.4. Example 4: A Phase 2a, Open-Label, Multicenter Study to Confirm the Efficacy and Safety of Obicetrapib in Participants with Early Alzheimer's Disease (Hetero/Homozygote E4 Carriers)

### 6.4.1. Study Design and Duration

This study is a multi-center, proof of concept. Phase 2a study in participants with early AD to confirm the efficacy, safety, and tolerability of obicetrapib therapy. Study duration for individual participants is approximately 24 weeks (excluding Screening and Follow-up).

### 6.4.2. Objectives

One objective of this study is to confirm the effect of obicetrapib in participants with early Alzheimer's Disease (AD) on levels of ApoA-1, ApoE, and cholesterol efflux capacity in cerebrospinal fluid (CSF). Another objective is to confirm the safety and tolerability of obicetrapib in participants with early AD. A further objective is to confirm the following:
- To confirm the effect of obicetrapib in participants with early AD on lipoprotein profiles (pharmacodynamics [PD]).
- To confirm the effect of obicetrapib in participants with early AD on biomarkers involved with neurodegeneration and inflammation.
- To confirm the effect of obicetrapib in participants with early AD on other PD exploratory biomarkers.
- To confirm the efficacy of obicetrapib in participants with early AD in delaying disease progression compared to standard of care.
- To confirm the efficacy of obicetrapib in participants with early AD on cognition.

### 6.4.3. Inclusion Criteria

Participants who meet all of the following criteria are eligible to participate in the study:
1. Age range: 50-75 years of age at the Screening Visit
2. Males, or females who are post-menopausal or otherwise not of child-bearing potential
3. Diagnosis of AD based on the NIA-AA Research Framework criteria:
   a. Biomarker classification A+ T+N+ or A+T+N- based upon:
   i. CSF profile consistent with AD (an Aβ42 concentration of <1000 pg/mL AND p-tau >19 pg/mL, or a ratio of p-tau/Aβ42 of ≥0.024 (Elecsys assay)) taken during the Screening period prior to the day of the first dose of study medication or,
   ii. Documented evidence of a CSF profile consistent with AD obtained within the previous 12 months, or
   iii. Documented amyloid positron emission tomography (PET) scan evidence acquired within the previous 12 months
4. AD Clinical Stage 3 or 4 based on the NIA-AA Research Framework criteria
   a. Have a mini-mental state examination (MMSE) score at screening and baseline >20 inclusive
   b. Clinical Dementia Rating scale-Sum of Boxes (CDR-SB) global score ≥0.5 and ≤1 with memory box score ≥1.0
5. Able to speak, read and write the local language fluently
6. Have an APOE genotype of E4/E4 or E3/E4
7. Patients should either be:
   a. Not treated with any approved treatments for AD with a reasonable expectation that, based on the course of illness, need for treatment is not imminent and the patient should not be initiated on treatment for the length of the study, or
   b. Stabilized on an approved medication(s) for the treatment of AD for at least 3 months prior to baseline. The dose of the AD treatment should remain the same after entering the study.
8. Patient and care partner are willing to consent to all study procedures

### 6.4.4. Exclusion Criteria

Participants who meet any of the following criteria are excluded from participation in the study:
1. Other than AD, neurologic or medical disorder which may impair cognition including: head trauma, seizure disorder, neurodegenerative disease, hydrocephalus, cerebral/spinal hematoma, inflammatory disease, central nervous system infection (e.g., encephalitis or meningitis), neoplasm, toxic exposure, metabolic disorder (including hypoxic or hypoglycemic episodes), or endocrine disorder, or any significant medical conditions that, in the opinion of the investigator, prohibits their participation in the study.
2. Any contra-indication to undergo magnetic resonance imaging (MRI), as judged by local Principal Investigator (PI) or radiologist.
3. MRI of the brain indicative of significant abnormality, including, but not limited to, prior hemorrhage or infarct >1 cm³, >3 lacunar infarcts, deep white matter lesions corresponding to a Fazekas score of 3, cerebral contusion, encephalomalacia, aneurysm, vascular malformation, subdural hematoma, hydrocephalus, space-occupying lesion (eg, abscess or brain tumor such as meningioma). Small incidental meningiomas may be allowed if discussed and approved by the Medical Monitor.
4. History of any of the following neurological, psychiatric or medical conditions:
   a. History of large vessel stroke
   b. History of myocardial infarction or unstable angina within the previous 12 months
   c. Or other clinical manifestations of atherosclerotic vascular disease
   d. Type 1 diabetes and uncontrolled type 2 diabetes (hemoglobin Alc [HbA1c] >8%)
   e. Systemic blood pressure >150/90 mmHg on 3 separate determinations
   f. History of hyperaldosteronism
   g. Significant renal or hepatic dysfunction
   h. Current or previous hepatitis B infection (defined as positive test for hepatitis B surface antigen (HbSAg) and/or hepatitis B core antibody (anti-HBc). Subjects with immunity to hepatitis B (if due to natural infection defined as negative HBsAg, positive hepatitis B antibody [anti-HBs] and positive anti-HBc; if due to vaccination defined as negative HBsAg, negative anti-HVc and positive anti-HBs) are eligible to participate in the study
   i. History or positive test at Screening for hepatitis C virus antibody (anti-HCV)
   j. History or positive test at Screening for human immunodeficiency virus (HIV)
   k. Diagnosed with cancer with metastatic potential within the last 5 years other than carcinoma in situ of the breast or cervix, or basal cell carcinoma of the skin that has been completely excised
   l. Major depressive episode requiring initiation of medication or hospitalization within the previous 90 days
   m. Presence of hallucinations or delusions
   n. Surgery within 12 weeks of screening
   o. For female subjects, pregnancy or ongoing lactation.
5. Any of the following laboratory abnormalities at Screening
   a. Clinically significant (as determined by a cardiologist or local PI) 12-lead ECG abnormalities
   b. Any serum chemistry value (e.g. aspartate aminotransferase [AST], alanine aminotransferase [ALT], alkaline phosphatase, creatine kinase [CK], total bilirubin etc) >2x the upper limit of normal (UIN) on 2 successive determinations less than 2 weeks apart
   c. Serum creatinine above the ULN or estimated glomerular filtration rate (eGFR) <60 mL/min
   d. Platelet cotuit, international normalized ratio (INR), prothrombin time (PT) or partial thromboplastin time (PTT) not within the normal range or other risk for increased or uncontrolled bleeding
   e. Not carrying an APOE 4 allele (e.g. E31E3; E3/E2; E2tE2).
6. Presence of contraindication to lumbar puncture as judged by local PI.
7. Any other significant medical conditions that in the opinion of the investigator, would prohibit participation in the study, including inability to tolerate the MRI scan or lumbar puncture procedures.
8. Taking any of the following medications
   a. Antipsychotic agents, including pimavanserin
   b. Stimulant medications
   c. Antidepressant medications whose dose has not been stable for at least 90 days
   d. Immunosuppressant medications, including chronic corticosteroids
   e. Injected or infused antibody therapies, including but not limited antibodies directed against tumor necrosis factor (TNF), anti-interleukin(IL)-6, natalizumab, rituximab and similar agents
   f. Insulin
   g. Anticoagulant or anti-platelet medications including warfarin, heparinoids and direct coagulation factor inhibitors (e.g. apixaban, dagibatran, rivaroxaban)
      *either* aspirin at a dose of <100 mg/day or clopidogrel at a dose of 75 mg/day, *but not both in combination* is permitted
9. Participation in any other interventional clinical trial, or treatment with any investigational drug or investigational use of an approved therapy within 30 days (or 5 half-lives of such agent) prior to the first Screening visit.
10. Regular use of cannabis or cannabis products, including non-prescription products containing cannabidiol (CBD)
11. History of drug (including cannabis) or alcohol abuse within the last 5 years

### 6.4.5. Retesting

If laboratory abnormalities during Screening are considered by the Investigator to be transient, then the laboratory tests may be repeated once during Screening. The Investigator's rationale for retesting should be documented. If the retest result is no longer exclusionary, the participant may be enrolled.

### 6.4.6. Rescreening

Participants who have screen-failed are permitted to rescreen once, following consultation with the Medical Monitor. Rescreening may be scheduled after at least 5 days have elapsed from the previous study visit.

### 6.4.7. Withdrawal Criteria

Participation in this clinical study may be discontinued for any of the following reasons:
1. The participant withdraws consent or requests discontinuation from the study for any reason;
2. Occurrence of any medical condition or circumstance that exposes the participant to substantial risk and/or does not allow the participant to adhere to the requirements of the protocol;
3. Any SAE, clinically significant adverse event (AE), severe laboratory abnormality, intercurrent illness, or other medical condition which indicates to the Investigator that continued participation is not in the best interest of the participant;
4. Pregnancy;
5. Requirement of prohibited concomitant medication;
6. Participant failure to comply with protocol requirements or study-related procedures; or
7. Termination of the study by the Sponsor or the regulatory authority.

If a participant withdraws prematurely from the study due to the above criteria or any other reason, study staff should make every effort to complete the full panel of assessments scheduled for the Early Termination Visit. The reason for participant withdrawal must be documented in the electronic case report form (eCRF).

In the case of participants lost to follow-up, at least 3 attempts to contact the participant must be made and documented in the participant's medical records. Withdrawal participants will not be replaced.

### 6.4.8. Study Treatments

Participants are treated with obicetrapib 10 mg per day.

### 6.4.8.1 Rationale for Dosing

In previous multiple-dose clinical studies of obicetrapib in healthy subjects and patients, near maximal effects were observed with the 5 mg obicetrapib dose. At this dose level, CETP activity and concentrations were effectively reduced, and HDL-C levels were increased while LDL-C levels decreased.

### 6.4.8.2 Randomization and Blinding

Participants who meet all eligibility criteria are enrolled into the study. All participants are treated with Obicetrapib 10 mg. Breaking the blind is not applicable, as this an open-label study.

### 6.4.8.3 Drug Supplies

### 6.4.8.3.1 Formulation and Packaging

The study drugs consist of 5 mg obicetrapib tablets. All products are manufactured in accordance with current European Union Good Manufacturing Practice.

Obicetrapib tablets are round, white film-coated tablets, with no identifying markings, containing 5 mg of obicetrapib calcium drug substance. The excipients present in the tablet cores are microcrystalline cellulose, mannitol, sodium starch glycollate, colloidal silicon dioxide, and magnesium stearate. A commercially available film-coating formula (Opadry II white, ex Colorcon) is applied to the cores.

Obicetrapib tablets are packaged into foil blisters and assembled into blister cards. The blister cards are clearly labelled to indicate which blisters to use on each day. Blister cards are assembled into kits, and each kit provides a sufficient supply for the necessary dosing. The shelf-life is assigned based on the stability of the individual products. The kits should be stored below 25°C.

The physical, chemical, and pharmaceutical formulation properties and characteristics of the obicetrapib tablets are described in the Investigator's Brochure.

All study drugs are labelled in accordance with all applicable local regulatory requirements.

### 6.4.8.3.2 Study Drug Preparation and Dispensing

The study drugs used in this study are 5 mg obicetrapib tablets.

At each appropriate visit, participants receive a kit containing blister cards with the study drug appropriate for the participant's treatment group. Participants are instructed to take 2 tablets from the blister cards in the kit each day. The blister cards are clearly labelled to indicate which blisters to use on each day. Each kit provides a sufficient supply for 1 month of dosing. Participants are instructed to return all unused study drugs at the next visit.

### 6.4.8.3.3 Study Drug Administration

Study drugs are administered by the participant orally and once daily on Days 1 through 126. Study drugs should be administered at approximately the same time each morning, with food. On days with visits scheduled, study drugs should be administered with food following all fasted blood samples. If a participant forgets to take study drug on a given day, they should take the next dose as normal and should not take a double dose to make up for the forgotten dose.

### 6.4.8.3.4 Treatment Compliance

Compliance to the study drug regimen is evaluated by counting unused tablets and capsules. During the Treatment Period, if compliance is not between 80% and 120%, inclusive, the participant is counselled about the importance of compliance to the regimen. If the limits are exceeded at 2 consecutive visits, a decision is made by the Investigator and Sponsor as to whether the participant should be withdrawn from the study.

### 6.4.8.3.5 Storage and Accountability

All study drugs are stored below 25°C in a secure area with access limited to the Investigator and authorized site personnel.

In accordance with regulatory requirements, the Investigator or designated site personnel must document the amount of study drug dispensed and/or administered to participants, the amount returned by participants, and the amount received from and returned to the Sponsor (or representative) when applicable. Study drug accountability records must be maintained throughout the course of the study. Discrepancies are to be reconciled or resolved. Procedures for final disposition of unused study drug are provided in the appropriate study manual.

### 6.4.9. Prior and Concomitant Medications and/or Procedures

### 6.4.9.1 Excluded Medications and/or Procedures

Any lipid-altering therapy other than the investigational study drug is prohibited during the study.

### 6.4.9.2 Documentation of Prior and Concomitant Medication Use

Medications used within 28 days prior to the Screening Visit are recorded. Any medications administered in addition to the study drugs, whether allowed per the protocol or not, must be documented on the concomitant medication eCRF.

### 6.4.10. Study Procedures

Study procedures follows the Schedule of Procedures:

| **Study period** | **Screening** | **Treatment Period** | | | | | **Follow-Up** |
|---|---|---|---|---|---|---|---|
| | | **Baseline (VI)** | **V3** | **V4** | **V5** | **EOT/ET⁶ (V5)** | |
| **Day (±Visit Window)** | **1-8 weeks** | **D1** | **D42 ±7d** | **D84 ±7d** | **D126 ±7d** | **D168 ±7d** | **D198** |
| Informed consent | X | | | | | | |
| Inclusion/exclusion criteria | X | X | | | | | |
| Demographic data | X | | | | | | |
| Medical historv and baseline conditions | x | | | | | | |
| Height and weight | X | | | | | X | |
| Vital signs | X | | X | X | X | X | |
| ECG | X | | | | | X | |
| Pregnancy test (if applicable) | X | | | | | | |
| Thyroid function | X | | | | | | |
| Folic acid and vitamin B12 levels | X | | | | | | |
| Hematology, chemistry and urinalysis | X | | | | | X | |
| Coagulation | X | | | | | | |
| APOE E4 testing | X | | | | | | |
| MRI-cerebrum | X | | | | | | |
| Physical examination | X | | | X | | X | |
| Neurological examination | X | | | X | | X | |
| Prior and concomitant medications | X | X | X | X | X | X | |
| Dispense study drugs | | X | X | X | X | | |
| Study drug administration | | X | X | X | X | X | |
| Study drug compliance check | | | X | X | X | X | |
| Adverse events | X | X | X | X | X | X | |
| CDR-SB | X | X | | | | X | |
| MMSE | X | X | | X | | X | |
| MOCA | | X | | | | X | |
| CFC | | X | | | | X | |
| Plasma sample for biomarkers | | X | | X | | X | |
| CSF sample for biomarkers | X | | | X⁷ | | X | |

| | | | | | | | |
|---|---|---|---|---|---|---|---|
| APOE E4 = apolipoprotein E-E4; CDR-SB = Clinical Dementia Rating scale-Sum of Boxes; CFC = Cognitive-Functional Composite; ECG = electrocardiogram; EOT = End of Treatment; ET = Early Termination; MMSE = mini-mental state examination; MOCA = Montreal Cognitive Assessment; MRI = magnetic resonance imaging. | | | | | | | |

### 6.4.10.1.1 Clinical Laboratory Evaluations

Blood for chemistry and hematology are obtained as indicated in the Schedule of Procedures and sent to a central laboratoty for analysis. In particular, the following lipid profile data are collected:
Apolipoprotein B
Apolipoprotein E (ApoE)
High-density lipoprotein-ApoE [1]
High-density lipoprotein cholesterol
Low-density lipoprotein cholesterol [2]
Non-high-density lipoprotein cholesterol
Triglycerides
Very low-density lipoprotein cholesterol
Apolipoprotein Al
Apolipoprotein All

[note 1] With and without apolipoprotein C3.
[note 2] Measured by preparative ultracentrifugation, also referred to as beta quantification.

As indicated in the Schedule of Procedures, the following are among the AD biomarker profile data collected in the CSF and/or plasma:
Tau and pTau epitopes
Aβ₁₋₄₂, Aβ₁₋₄₀, Aβ₁₋₄₂/Aβ₁₋₄₀ ratio
Neurogranin
Neurofilament light
GFAP (glial fibrillary acidic protein)
YKL40
PDGFRB.

Blood samples for chemistry and hematology must be obtained under fasting conditions (i.e., after the participant has fasted for ≥10 hours). For the purposes of this study, fasting is defined as nothing by mouth except water and any essential medications. If a participant is not fasting, the Investigator is to reschedule the visit as soon as possible. Estimated glomerular filtration rate is calculated using the Chronic Kidney Disease Epidemiology Collaboration equation. At the Screening Visit only, the hematology panel includes HbA1c, TNR, PT and PTT.

Urine is obtained as indicated in the Schedule of Procedures and is sent to a central laboratory for complete urinalysis.

A urine pregnancy test is performed only prior to participation in the study at the screening visit.

### 6.4.10.1.2 Electrocardiograms

A single, standard 12-lead ECG is performed by the Investigator or trained site personnel at the Screening Visit and centrally read.

### 6.4.10.1.3 Physical Examinations

A physical examination is performed as indicated in above Schedule of Procedures.

### 6.4.11. Efficacy Assessments

The primary efficacy endpoint is the change from baseline in levels of ApoA-I, ApoE, and cholesterol efflux capacity in CSF, measured at week 24.

Among secondary efficacy endpoints specific to lipid profile measurement in CSF and plasma, are:
- percent change of HDL-C at Screening (Visit 1) or Baseline (Visit 2), at Day 84 (Visit 4), and at the End of Treatment (Day 168, Visit 6).
- percent change of HDL subfractions at Screening (Visit 1) or Baseline (Visit 2), at Day 84 (Visit 4), and at the End of Treatment (Day 168, Visit 6).
- percent change of HDL-ApoE in HDL particles lacking ApoC3 at Screening (Visit 1) or Baseline (Visit 2), at Day 84 (Visit 4), and at the End of Treatment (Day 168, Visit 6).
- percent change of ApoA-II at Screening (Visit 1) or Baseline (Visit 2), at Day 84 (Visit 4), and at the End of Treatment (Day 168, Visit 6).
- percent change of 24S-hydroxycholesterol and 27-hydrox-ycholesterol at Baseline (Visit 2), at Day 84 (Visit 4) and the End of Treatment (Visit 6).

Additional secondary efficacy endpoints specific to AD biomarkers in CSF:
- percent change of Tau and pTau epitopes at Screening (Visit 1), at Day 84 (Visit 4), and at the End of Treatment (Day 168, Visit 6).
- percent change of Aβ₁₋₄₂, Aβ₁₋₄₀, Aβ₁₋₄₂/Aβ₁₋₄₀ ratio at Screening (Visit 1), at Day 84 (Visit 4), and at the End of Treatment (Day 168, Visit 6).
- percent change of neurogranin at Screening (Visit 1), at Day 84 (Visit 4), and at the End of Treatment (Day 168, Visit 6).
- percent change of neurofilament light at Screening (Visit 1), at Day 84 (Visit 4), and at the End of Treatment (Day 168, Visit 6).
- percent change of GFAP at Screening (Visit 1), at Day 84 (Visit 4), and at the End of Treatment (Day 168, Visit 6).
- percent change of YKL40 at Screening (Visit 1), at Day 84 (Visit 4), and at the End of Treatment (Day 168, Visit 6).
- percent change of PDGFRb at Screening (Visit 1), at Day 84 (Visit 4), and at the End of Treatment (Day 168, Visit 6).

Additional secondary efficacy endpoints specific to AD biomarkers in plasma are:
- percent change of Tau and pTau epitopes at Baseline (Visit 2), at Day 84 (Visit 4), and at the End of Treatment (Day 168, Visit 6).
- percent change of Aβ₁₋₄₂, Aβ₁₋₄₂, Aβ₁₋₄₂ ratio at Baseline (Visit 2), at Day 84 (Visit 4), and at the End of Treatment (Day 168, Visit 6).
- percent change of neurogranin at Baseline (Visit 2), at Day 84 (Visit 4), and at the End of Treatment (Day 168, Visit 6).
- percent change of neurofilament light at Baseline (Visit 2), at Day 84 (Visit 4), and at the End of Treatment (Day 168, Visit 6).

Additional secondary efficacy endpoints to track disease progression are:
- administer the Cognitive-Functional Composite test at Baseline (Visit 2) and End of Treatment (Day 168, Visit 6).
- administer the CDR-SB test at Screening (Visit 1) and End of Treatment (Day 168, Visit 6).
- administer the Mini-Mental State Examination at Screening (Visit 1), Baseline (Visit 2), Day 84 (Visit 4) and End of Treatment (Day 168, Visit 6).

### 6.4.12. Results

The administration of obicetrapib is effective by End of Treatment to increase plasma levels of total ApoE, HDL-ApoE, HDL-ApoE in particles without Apo-C3, and the percentage of HDL-ApoE in particles lacking ApoC3 in patients who have early AD and at least one ApoE4 allele.

The administration of obicetrapib is effective by End of Treatment to prevent at least one of (i) further decrease in levels of Aβ42 in plasma, (ii) further increase in levels of Aβ40 in plasma, (iii) further decrease in ratio of Aβ42/Aβ40 in plasma, (iv) further increase in levels of neurofilament light (NFL) in plasma, and (v) further increase in levels of neurogranin in plasma, as compared to levels immediately prior to start of treatment.

The administration of obicetrapib is effective to prevent at least one of (i) further decrease in levels of Aβ42 in CSF, (ii) further increase in levels of Aβ40 in CSF, (iii) further decrease in ratio of Aβ42/Aβ40 in CSF, (iv) further increase in levels of neurofilament light (NFL) in CSF, and (v) further increase in levels of neurogranin in CSF, as compared to levels immediately prior to start of treatment.

Subjects experience no significant decline in cognitive status between enrollment and End of Treatment.

### 7. EQUIVALENTS AND INCORPORATION BY REFERENCE

While the invention has been particularly shown and described with reference to a preferred embodiment and various alternate embodiments, it will be understood by persons skilled in the relevant art that various changes in form and details can be made therein without departing from the spirit and scope of the invention.

All references, issued patents and patent applications cited within the body of the instant specification are hereby incorporated by reference in their entirety, for all purposes.

## Claims

1. A method of slowing progression of neurodegenerative disease in a subject who has or is at risk of developing a neurodegenerative disease, comprising:
administering a therapeutically effective amount of obicetrapib or a pharmaceutically acceptable salt thereof to the subject.

2. The method of claim 1, wherein obicetrapib or salt thereof is administered orally.

3. The method of claim 1 or claim 2, wherein obicetrapib is administered once per day.

4. The method of claim 3, wherein the dose is 5-25 mg po QD of obicetrapib or salt thereof.

5. The method of claim 4, wherein the dose is 5 mg po QD.

6. The method of claim 4, wherein the dose is 10 mg po QD.

7. The method of claim 4, wherein the dose is 15 mg po QD.

8. The method of claim 4, wherein the dose is 20 mg po QD.

9. The method of claim 4, wherein the dose is 25 mg po QD.

10. The method of any one of claims 1-9, wherein obicetrapib or salt thereof is administered once daily for at least 8 weeks.

11. The method of claim 10, wherein obicetrapib or salt thereof is administered once daily for at least 6 months.

12. The method of claim 11, wherein obicetrapib or salt thereof is administered once daily for at least 12 months.

13. The method of claim 12, wherein obicetrapib or salt thereof is administered once daily for at least 24 months.

14. The method of any one of claims 1-13, wherein the subject has been diagnosed with or is at risk for Alzheimer's Disease (AD), Lewy Body dementia, vascular or multi-infarct dementia, or frontotemporal dementia (FTD).

15. The method of claim 14, wherein the subject has been diagnosed with or is at risk for developing AD.

16. The method of claim 15, wherein the subject has been diagnosed with AD.

17. The method of claim 15 or claim 16, wherein the subject has at least one ApoE4 allele.

18. The method of claim 17, wherein the subject has two ApoE4 alleles.

19. The method of any one of claims 17-18, further comprising the prior step of detecting ApoE4 alleles in the subject.

20. The method of any one of claims 1-19, wherein the subject has been diagnosed with mild cognitive impairment (MCI).

21. The method of any one of claims 1-20, wherein the subject has been determined, prior to treatment, to have at least one of (i) decreased levels of Aβ42 in plasma, (ii) increased levels of Aβ40 in plasma, (iii) decreased ratio of Aβ42/Aβ40 in plasma, (iv) increased levels of neurofilament light (NfL) in plasma, and (v) increased levels of neurogranin in plasma, as compared to a healthy control population that does not have and that is not at elevated risk for neurodegenerative disease.

22. The method of any one of claims 1-21, wherein the subject has been determined, prior to treatment, to have at least one of (i) decreased levels of Aβ42 in CSF, (ii) increased levels of Aβ40 in CSF, (iii) decreased ratio of Aβ42/Aβ40 in CSF, (iv) increased levels of neurofilament light (NFL) in CSF, and (v) increased levels of neurogranin in CSF, as compared to a healthy control population that does not have and that is not at elevated risk for neurodegenerative disease.

23. The method of any one of claims 1-22, wherein the subject has been determined, prior to treatment, to have abnormal patterns of Tau based on Tau PET imaging.

24. The method of any one of claims 1-23, wherein the dose of obicetrapib or salt thereof is sufficient to prevent at least one of (i) further decrease in levels of Aβ42 in plasma, (ii) further increase in levels of Aβ40 in plasma, (iii) further decrease in ratio of Aβ42/Aβ40 in plasma, (iv) further increase in levels of neurofilament light (NFL) in plasma, and (v) further increase in levels of neurogranin in plasma, as compared to levels immediately prior to start of treatment.

25. The method of any one of claims 1-24, wherein the dose of obicetrapib or salt thereof is sufficient to prevent at least one of (i) further decrease in levels of Aβ42 in CSF, (ii) further increase in levels of Aβ40 in CSF, (iii) further decrease in ratio of Aβ42/Aβ40 in CSF, (iv) further increase in levels of neurofilament light (NFL) in CSF, and (v) further increase in levels of neurogranin in CSF, as compared to levels immediately prior to start of treatment.

26. The method of any one of claims 1-25, wherein obicetrapib is administered as a tablet.

27. The method of claim 26, wherein the tablet comprises obicetrapib as the calcium salt.

28. Obicetrapib, or a pharmaceutically acceptable salt thereof, for use in the treatment of slowing the progression of neurodegenerative disease, wherein the subject has or is at risk of developing a neurodegenerative disease.

29. Obicetrapib, or a pharmaceutically acceptable salt thereof, for use in the manufacture of a medicament for the treatment of slowing the progression of neurodegenerative disease, wherein the subject has or is at risk of developing a neurodegenerative disease.
